# EUROPEAN PATENT SPECIFICATION

(11) **EP 1 590 041 B1**
(45) Date of publication and mention of the grant of the patent: **01.07.2015**
(21) Application number: 03754493.9
(22) Date of filing: 11.09.2003
(51) Int. Cl.: A61K 9/00, A61N 1/30, A61K 41/00

(54) **TREATMENT OF PERIODONTAL DISEASE WITH PHOTOSENSITIZERS**
BEHANDLUNG VON ZAHNFLEISCHERKRANKUNG MIT PHOTOSENSIBILISATOREN
TRAITEMENT DE PARODONTOPATHIES AU MOYEN D'AGENTS PHOTOSENSIBILISANTS

(30) Priority: 12.09.2002 US 241958
(43) Date of publication of application: 02.11.2005
(73) Proprietor: Biolitec Pharma IP & Investment Ltd., 87018 Labuan, F.T. Labuan (MY)
(72) Inventor: NEUBERGER, Wolfgang, 87000 F.T. Labuan (MY)
(74) Representative: Herrmann, Franz
(86) International application number: PCT/US2003/028455
(87) International publication number: WO 2004/024080

(56) References cited:
- EP-A1- 0 451 390
- WO-A1-01/00248
- WO-A1-98/39011
- WO-A2-02/45575
- JP-A- 2001 114 658
- US-A- 5 611 793
- US-A- 6 056 548
- DATABASE BIOSIS REVIEWS (FILE 5) [Online] BHATTI M. ET AL.: 'Antibody-targeted lethal photosensitization of Porphyromonas gingivalis', XP002977565 Retrieved from DIALOG Database accession no. 200000499391 & ANTIMICROBIAL AGENTS AND CHEMOTHERAPY vol. 44, no. 10, October 2000, pages 2615 - 2618
- ANONYMUS: 'Photosensitizing agents', [Online] 07 February 2011, page 1 NCBI Mesh Retrieved from the Internet: <URL:htttp:/www.ncbi.nlm.nih.gov/mesh/68017 319> [retrieved on 2011-02-07]
- GRANT W E ET AL: "Photodynamic therapy of oral cancer: photosensitisation with systemic aminolaevulinic acid", THE LANCET, LANCET LIMITED. LONDON, GB, vol. 342, 17 July 1993 (1993-07-17), pages 147-148, XP002132811, ISSN: 0140-6736, DOI: 10.1016/0140-6736(93)91347-O

## Description

### Background of the invention

### 1. Field of the invention

The present invention relates to the use of photosensitizing compounds in long term effect or timed release formulations and laser irradiation to kill the microbes involved in a number of oral diseases including inflammatory periodontal disease.

### 2. Information Disclosure Statement

Advanced periodontal disease is one of a large number of oral infectious diseases, and is the principal cause of tooth loss in those over 30 years old. The term "periodontal disease" actually encompasses a number of diseases that affect the supporting tissues of the teeth. The periodontium consists of the investing and supporting tissues of the tooth, and consists of the gingiva, the periodontal ligament, the cementum and the alveolar bone. The periodontium is subject to morphological and functional variations associated with age and pathology. The term gingivitis refers to an inflammation of the gingival tissue. Recent research on periodontal disease shows that not all patients with gingivitis progress to periodontal disease. However, gingivitis may be the first sign of oncoming periodontal disease and most, if not all, periodontal disease features accompanying gingivitis. Periodontitis is defined as inflammation involving the gingival unit (gingiva and alveolar mucosa) and extends to the periodontal ligament, alveolar bone, and cementum. Periodontitis is characterized by loss of clinical attachment of the gingiva and radiographic loss of bone.

The vast body of evidence indicates that the primary cause of both gingivitis and periodontitis is bacterial activity. Bacteria attach to the tooth surface at and slightly under the gingival margin. They colonize and form an organized mass that is referred to as bacterial plaque. This plaque, if allowed to remain, brings about inflammatory changes in the tissues. The pathogenic potential of plaque can vary from one individual to another and from tooth to tooth within an individual. The reaction of the host tissues to this bacterial attack is through an inflammatory and immunologic defense mechanism. Periodontal diseases arise from the interaction between bacterial cells and their products in dental plaque and the host defense mechanisms.

A variety of treatment modalities have been utilized to combat the disease and prevent its progression. Periodontal surgery is one such modality, and it includes curettage, gingivectomy, and flap or pocket elimination including the modified Widman flap procedure. Chemotherapy is also used, particularly local and systemic antibiotic treatment. The antibiotic agents most frequently used in chemotherapy for periodontal disease are the tetracyclines and metronidazole.

The conventional methods described above suffer from a number of significant drawbacks. Surgical reduction of periodontal pockets can expose roots, cause sensitivity, and result in poor aesthetics by lengthening the clinical crown and altering the gingival contours. Surgical treatment does not guarantee that the disease process will not recur to the detriment of the patient's dentition. It is reported that to treat periodontitis the effective concentration of antibiotics such as minocycline HCl must be maintained for at least 7 to 10 days.

According to U.S. Patent No. 5,599,553, it is reported that a pharmaceutical preparation composed of minocycline HCl and polycaprolactone in the form of a delivery film can release the active substance for 7 days in the periodontal pocket. However, in this case polycaprolactone must be completely removed after the drug is released because it takes too long to decompose in the body. This problem can be overcome by a formulation for treating periodontitis using biodegradable microspheres which retain an effective drug concentration in the periodontal pocket for a certain time after a single dose. There is no discomfort due to the feeling of a foreign substance because the formulation is prepared with microparticles, and there is no need to remove the formulation after treatment because it is biodegradable. However, these improvements do not address the need for repeated treatments, since the dental disease is generally chronic.

However, antibiotic therapy is usually not totally successful, particularly in advanced cases, probably because the site of action of periodontal disease is not accessible to a sufficient concentration of drug at the base of the periodontal pocket to completely halt the disease process. Moreover, the long-term use of antimicrobial agents, such as chlorhexidine and tetracycline, promote the development of resistance which render the agents clinically ineffective and can also disturb the oral microflora. Anti-microbial solutions have been introduced through irrigating devices to the periodontal pocket with some success, but are not curative. Sustained systemic administration of antibiotics such as tetracyclines is inadvisable because of the potentially serious side effects and the possibility of giving rise to antibiotic-resistant infections. New, more effective approaches to the treatment of periodontal disease, particularly periodontitis, are actively being sought.

Photodynamic therapy (PDT) has been proposed as an attractive method of eliminating oral bacteria and bacteria in topical and gastrointestinal infections because these sites are relatively accessible to illumination. For example, Wilson and Harvey proposed in U.S. Patent No. 5,611,793 the use of photosensitizers in combination with suitable irradiation to treat several oral diseases. However, this method suffers from the fact that the duration of the treatment is not sufficient. To treat periodontitis, effective concentration of the active substance in the periodontal pocket must be maintained for a relatively long time.

WO 9839011 A1 discloses a method of treatement of a disorder characterized by the presence of an unwanted bacterium in the oral cavity, comprising administering a photosensitizer conjugate and providing iridiation to produce a phototoxic effect by the photosensitizer.

It would be useful to provide a method of treatment for periodontitis which overcomes the drawbacks of prior art methods and further may be practiced or continued by patients at home as well as utilized by professional dental practitioners. The present invention address this need.

### Brief Summary and Objectives of the Invention

The present invention is directed to a time-release photosensitizer composition according to claim 1.

It is an object of the present invention to provide a method for treatment of oral diseases characterized by long duration.

It is another object of the present invention to use photodynamic therapy (PDT) for the treatment of oral diseases with photosensitizers applied in a timed release formulation or coating at the desired sites.

It is a further object of the present invention to activate the photosensitizers by irradiation at suitable wavelengths with light, applied either by the dentist or as repeated treatment by the patient at home e.g. using a laser toothbrush.

It is a still further object of the present invention to modify the photosensitizer to more effectively target the photosensitizer to the unwanted (bacterial) species.

Briefly stated, the present invention discloses a method and composition for treating oral diseases, including inflammatory periodontal disease, by utilizing photosensitizing compounds in long term effect or timed release formulations, including local highly concentrated formulations in biofilms on teeth and implants and oral wounds as well as periodontal pockets, and activating the photosensitizers with radiation to selectively destroy bacteria and other microbial bodies. Photosensitizers are incorporated in a timed release formulation characterized by release of photosensitizers over a prolonged period of time. The photosensitizers are housed within micro- or nanoparticles, and can be gradually released through biodegradation or periodically released by such processes as brushing, irradiation and chemically induced release. The diffusion speed of the photosensitizers can be accelerated by brushing. The formulation is applied to the oral cavity and allowed to settle on biofilms therein, in periodontal pockets formed by the disease, or coated at the desired sites. Released photosensitizers are activated by radiation of preselected wavelengths in one or repeated treatments which can be carried out either by a dental professional or as a repeated treatment by the patient at home with, for example, a laser toothbrush. In another embodiment, the photosensitizer molecule is also modified to more effectively target the molecule to the unwanted oral bacterial species. The present invention has further use in that the micro- or nanoparticles act to protect photosensitizers from inactivation or degradation by saliva or other body fluids.

The above, and other objects, features and advantages of the present invention will become apparent from the following description.

### Detailed Description of Preferred Embodiments

The present invention provides a composition for disinfecting or sterilizing tissues in the oral cavity or a wound or lesion in the oral cavity. Also disclosed is a method that comprises applying a photosensitizing compound in a timed release formulation, as well as a formulation which provides locally high concentrations of primary protected photosensitizers, to the tissues, wound or lesion. Photosensitizers that produce highly reactive singlet oxygen upon activation by irradiation are known to be effective in destroying unwanted (bacterial) species and thereby disinfecting and sterilizing lesions and wounds as well as implants in the oral cavity. The timed release is characterized by providing a sustained concentration of the photosensitizer in or around a treatment area during a prolonged time period in which the photosensitizer can be activated one or several times by irradiating the tissues, wound or lesion with laser light at a wavelength absorbed by the photosensitizing compound.

This long term formulation is characterized by its ability to maintain a certain concentration of the photosensitizer drug over a prolonged period of time, and it thus provides the ability to repeatedly activate the photosensitive compound after a single application and thereby achieve healing due to repeated treatments over an extended time period with only one application of the formulation. The photosensitive molecules are included in and released from micro- or nanoparticles by controlled diffusion. Biodegradable nanoparticles can be used to avoid the step of actively releasing the molecules. In that case, the duration of the timed release would be limited and would vary depending on the composition of the nanoparticles. In another embodiment, release from some or all of the micro- or nanoparticles can be actively and controllably induced by the user or practitioner as part of the treatment.

These formulations are especially useful in treating chronic diseases like periodontal lesions where repeated treatments are necessary to achieve healing. After a photosensitizer in a timed release formulation is administered to the affected area, it is left in contact with the microbes for a period of time to enable the microbes to take up some of the photosensitizer and become sensitive to the laser light. The photosensitizers are subsequently activated by radiation at suitable wavelengths for one or more treatments to destroy the target microbes. The activation can be carried out by a dental practitioner or even by the patient at home with, for example, a laser toothbrush.

In preferred aspects of the invention, the described treatment with photosensitizing compounds and laser irradiation can be applied to a variety of tasks including:
the destruction of disease-related microbes in a periodontal pocket in order to treat chronic periodontitis;
the destruction of disease-related microbes in the region between the tooth and gingiva (gingival crevice or gingival margin) in order to treat or prevent inflammatory periodontal diseases including chronic periodontitis, gingivitis and the like; and
the disinfection or sterilization of dental implants and dental and/or gingival tissues in other dental surgical procedures.

The advantage of the use of photosensitive compounds over other methods, such as antibiotic treatments, is the ability to controllably activate the compound with radiation and therefore enhance treatment effects. The long term use of antibiotics often promotes the development of resistance rendering the agents clinically ineffective and also disturbs the oral microflora. Additionally, high concentrations of antibiotics may have undesired side effects in the tissue and on the oral microflora. Since the photosensitive compound shows no toxicity without irradiation, the application of high concentrations and short and possibly repeated activation times show enhanced therapeutic effects and reduced side effects. High concentrations of the photosensitizer can be achieved, for example, by the use of specialized micro- and nano-particles such as dendrimers. Moreover, the ability of the compound to destroy only the unwanted species and leave healthy tissue intact can be enhanced by modifying the compound with targeting moieties. These modifications can affect chemical or physical properties that show an affinity to areas such as the cell membranes of unwanted bacterial species. Other targeting methods including coupling with antibodies or other affinity molecules are conceivable.

It is preferred that the photosensitizers used in the method of the present invention are activatable by light at the red end of the visible spectrum or at longer wavelengths, preferably between 630 and 800 nm. Light at these wavelengths is better able to penetrate tissues surrounding a wound or lesion, such as oral tissues, and, in particular, blood which may be present in the sites to be treated. This may be particularly useful in treating Alcove-like regions having honeycomb-like pockets. Several "second generation" photosensitive compounds with these spectral absorption properties are known to date, and include porphyrins, porphyrin derivatives, chlorins, pheophorbides, bacteriopheophorbides, phthalocyanines, naphthalocyanines, thiazines, xanthenes, pyrrylium dyes, psoralens, quinones. Also disclosed are photosensitizer precursors such as amenolevulic acid (ALA).

The timed release characteristic of the present formulation is due to the inclusion of polymeric micro- or nanoparticles, constructed with polymers, that contain the photosensitizer molecules. Release of photosensitizers from these polymers can occur by two general mechanisms. The first mechanism is diffusion resulting in the gradual release of the photosensitive molecules from the implant surface. The second mode of release occurs by the cleavage of the polymer backbone, defined as bulk erosion. Release can also be induced by a number of other processes. Such processes include radiation, ultrasonics, agitation through brushing, and chemical release such as with a solvent.

The micro- or nanoparticles consist of molecules building up polymers that are tolerable and non-toxic to the human or animal body. Several such compositions are known to date, among them are biodegradable and non-degradable compositions. Non-degradable particles must be removed after a certain time and thus the duration of the deposit effect can be chosen. The life time of biodegradable compounds is limited but can be chosen in a certain range by varying the composition and size of the polymer. The molecular weight of the polymers has an important effect on the period of drug release and degradation of the microspheres. The products of degradation processes such as hydrolysis of the particles are biologically compatible and metabolizable moieties which are eventually removed from the body. Polymer biodegradation products are formed at a very slow rate, and hence do not affect normal cell function. Moreover, the deposit effect can be achieved by coating the affected areas such as teeth, implants or periodontal pockets with the photosensitizer.

In addition to the advantage of being able to house and gradually or periodically release photosensitizers, nanoparticles offer an additional benefit. Many photosensitizers suffer from significant inhibition in effectiveness from exposure to saliva, white blood cells, and other natural defenses in the mouth. This can prove a significant problem in maintaining an effective concentration of photosensitizers in the mouth for an extended period of time. Because photosensitizers are encased in nanoparticles before treatment, they are shielded from these bodily fluids and thus do not degrade as quickly as they would if left unprotected. Non-biodegradable micro- or nanoparticles serve to protect photosensitizers until the photosensitizers are released. Likewise, biodegradable nanoparticles also protect photosensitizers until they degrade to the point where they release the photosensitizers. In this way, micro- or nanoparticles further serve to prolong the period of time that photosensitizers can remain in the oral cavity.

It should be noted that the photosensitizer has to be in a certain concentration at the site of treatment and, for instance in the treatment of periodontal pockets and wounds where the treatment site may be flooded with body fluid such as saliva or blood, it might be necessary to apply the photosensitizers in greater concentration so as to achieve an effective concentration after dilution by the body fluid. Moreover, the treatment is more effective if the photosensitizer is applied in higher concentrations. These concentrations can be achieved by using highly specialized micro- and nanoparticles like dendrimer-photosensitizer complexes. Since the photosensitive compounds do not show any dark toxicity, these concentrations can be applied without side effects. Because exposure of the surrounding tissues of the oral cavity to the activated photosensitizer will generally be of short duration and highly localized, it may also be acceptable to use compounds which have some slight toxicity to these tissues.

It is a further object of the present invention to enhance the selectivity of the timed release photosensitizer towards unwanted species such as bacteria. The targeting is either achieved by chemically modifying the chemical or physical properties of the photosensitizer, or by coupling the molecule to a targeting moiety such as an antibody. It is generally preferred that the photosensitizer selected for use has a positive charge under physiological conditions since such photosensitizers are more readily taken up by the target microbes. Other targeting moieties that specifically recognize components of the bacterial cell membranes are known.

The present invention is further illustrated by the following examples, but is not limited thereby.

### Example 1: Bacterial redcution in biofilms within the oral cavity.

A dentist or patient can treat periodontal disease in various stages, or self-apply the treatment for prevention of diseases arising from microbes within the oral cavity.. A composition containing photosensitizers encased in micro- or nanoparticles is administered either by a dentist or by the patient. For patient application, a mouthwash is used containing a high concentration of the nanoparticles. The patient takes a prescribed dose of the mouthwash, and apply it as one would apply traditional mouthwashes to coat the mouth. Patient would then wait a prescribed period, typically 4 to 6 hours, or apply the mouthwash before bedtime. During this time, the mouth's natural saliva would serve to flush most of the micro- or nanoparticles from the mouth, leaving only those particle which are associated with the biofilms or trapped in existing periodontal pockets. After the prescribed period, the dentist/patient would then administer radiation using a laser toothbrush (as seen in, for example, U.S. Patent No. 5,658,148) to apply laser radiation for a prescribed duration or other means to direct irradiation to the treated sites within the oral cavity. The application would be similar in duration to the time taken to brush one's teeth. A prescribed frequency of about 2 times a day would effectively destroy harmful bacteria in the mouth.

### Example 2: Periodontal pocket treatment for disease.

A dental practitioner applies the photosensitizer formulation. A gel containing the above-described micor- or nanoparticles is applied by the dentist directly to the periodontal pockets. This is preferable for patients with more advanced stages of periodontal disease, in that the dentist can insure that sufficient photosensitizer reaches the deeper pockets. In one preferred embodiment the gel is chosen to harden after application, so that photosensitizer is set to slowly release over an extended period of time such as days/weeks etc. The pockets themselves should act to contain the photosenstizer for an extended period of time. The patient returns for periodic radiation treatments or in an alternative case self-applies radiation at prescribed intervals as described in Example 1 using a laser toothbrush, as mentioned above, or other suitable means.

## Claims

1. A time-release photosensitizer composition for use in the treatment of diseases of the oral cavity comprising the steps of:
a. administering a photosensitizer to an area needing treatment in a timed release formulation, wherein said timed release formulation controllably releases said photosensitizer to treatment sites; and
b. activating said photosensitizer by irradiating said area needing treatment at preselected wavelengths to reduce bacteria at that treatment area,
- wherein said photosensitizer is a photosensitive molecule selected from a group consisting of porphyrins, chlorins, pheophorbides, bacteriopheophorbides, phthalocyanines, naphthalocyanines, thiazines, xanthenes, pyrrylium dyes, psoralens, and quinones,
- further comprising micro- or nanoparticles that encase said photosensitizer, and wherein said timed release is achieved by controlled release of said photosensitizer from said micro- or nanoparticles,
- wherein said photosensitizer is activated by irradiating repeatedly at predetermined intervals, which range from a period of hours to days.

2. The composition for use in the treatment of diseases of the oral cavity according to claim 1,
for treating periodontal pockets.

3. The composition for use in the treatment of diseases of the oral cavity according to claim 1,
for treating bacterial biofilms on teeth, on implants or in wounds.

4. The composition for use in the treatment of diseases of the oral cavity according to claim 1,
wherein said micro- or nanoparticles are biodegradable particles.

5. The composition for use in the treatment of diseases of the oral cavity according to any of claims 1 to 4,
wherein said timed release is achieved by periodically actively releasing said photosensitizer from a portion of said micro- or nanoparticles, wherein said method of actively releasing is selected from a group consisting of bulk erosion, radiation, ultrasonics, agitation through brushing, biochemical/enzymatic release, and chemical release.

6. The composition for use in the treatment of diseases of the oral cavity according to claim 5,
wherein said releasing by chemical release is by applying a solvent.

7. The composition for use in the treatment of diseases of the oral cavity according to claim 1,
for said administering of said photosensitizer in a formulation selected from a group consisting of direct application, application with a mouthwash, application with a gel, and application with a hardening gel.

8. The composition for use in the treatment of diseases of the oral cavity according to claim 1,
wherein said photosensitizer is activated by an irradiating step at preselected wavelengths between 630 and 800 nm.

9. The composition for use in the treatment of diseases of the oral cavity according to claim 8,
wherein said timed release formulation comprises a concentration of photosensitizer which allows repeated irradiation at predetermined intervals without additional applications of said composition.

10. The composition for use in the treatment of diseases of the oral cavity according to any of claims 1 to 9,
wherein said photosensitizer is a known photosensitizer precursor and wherein said photosensitizer precursor is aminolevulinic acid.

11. The composition for use in the treatment of diseases of the oral cavity according to claim 4 or 5,
wherein said photosensitizer comprises micro-or nanoparticle complexes to achieve high concentrations of said photosensitizer at a treatment area which can be activated by one or more irradiations.

12. The composition for use in the treatment of diseases of the oral cavity according to claim 11,
wherein said micro- or nanoparticle complexes are dendrimer-photosensitizer complexes.

13. The composition for use in the treatment of diseases of the oral cavity according to any one of claims 1 to 3,
wherein said photosensitizer comprises a modified photosensitizer complex having antibodies attached to said photosensitizer complex.

14. The composition for use in the treatment of diseases of the oral cavity according to any one of claims 1 to 3,
wherein said photosensitizer comprises a modified photosensitizer complex having molecular fragments attached to said photosensitizer complex, able to target components of bacterial cell membrane.

## Patentansprüche

1. Eine Zusammensetzung von Photosensibilisatoren mit verzögerter Freisetzung für die Verwendung bei der Behandlung von Krankheiten des Mundraums mit den Verfahrensschritten:
a. Anwenden eines Photosensibilisators auf einem behandlungsbedürftigen Bereich in einer Formulierung mit verzögerter Freisetzung, wobei die Formulierung mit verzögerter Freisetzung den Photosensibilisator auf beherrschbare Weise an den Behandlungsstellen freisetzt; und
b. Aktivieren des Photosensibilisators durch Bestrahlen des behandlungsbedürftigen Bereichs mit vorbestimmten Wellenlängen, um Bakterien in diesem Behandlungsbereich zu reduzieren,
- wobei der Photosensibilisator ein photoempfindliches Molekül ist, das aus der Gruppe ausgewählt ist, die aus Porphyrinen, Chlorinen, Pheophorbiden, Bakteriopheophorbiden, Phthalocyaninen, Naphthalocyaninen, Thiazinen, Xanthenen, Pyrrylium Farbstoffen, Psoralenen und Chinonen besteht,
- wobei die Zusammensetzung ferner Mikro- oder Nanoteilchen aufweist, die die Photosensibilisatoren beinhalten und wobei die verzögerte Freigabe durch eine beherrschte Freigabe der Photosensibilisatoren aus den Mikro- und Nanoteilchen erreicht wird,
- wobei die Photosensibilisatoren durch wiederholtes Bestrahlen in vorherbestimmten Zeitintervallen aktiviert werden, die sich zwischen Stunden und Tagen erstrecken.

2. Zusammensetzung für die Verwendung bei der Behandlung von Krankheiten des Mundraums gemäß Anspruch 1,
zur Behandlung von parodontalen Taschen.

3. Zusammensetzung für die Verwendung bei der Behandlung von Krankheiten des Mundraums gemäß Anspruch 1,
zur Behandlung von bakteriellen Biofilmen auf Zähnen, auf Implataten oder in Wunden.

4. Zusammensetzung für die Verwendung bei der Behandlung von Krankheiten des Mundraums gemäß Anspruch 1,
bei der die Mikro- oder Nanoteilchen biologisch abbaubare Teilchen sind.

5. Zusammensetzung für die Verwendung bei der Behandlung von Krankheiten des Mundraums nach einem der Ansprüche 1 bis 4,
bei der die verzögerte Freigabe bewirkt wird, indem der Photosensibilisator aktiv periodisch aus einem Teil der Mikro- oder Nanoteilchen freigesetzt wird, wobei das Verfahren zur aktiven Freisetzung aus einer Gruppe ausgewählt ist, die aus Massenerosion, Strahlung, Ultraschall, Bewegen durch Bürsten, biochemische enzymatische Freisetzung und chemische Freisetzung besteht.

6. Zusammensetzung für die Verwendung bei der Behandlung von Krankheiten des Mundraums nach Anspruch 5,
bei der die durch eine chemische Freisetzung erfolgende Freisetzung eine Anwendung eines Lösungsmittels ist.

7. Zusammensetzung für die Verwendung bei der Behandlung von Krankheiten des Mundraums gemäß Anspruch 1,
zur Anwendung des Photosensibilisators in einer Formulierung, die aus einer Gruppe ausgewählt ist, die aus direkter Anwendung, Anwendung mit einer Mundwäsche, Anwendung mit einem Gel und Anwendung mit einem aushärtenden Gel besteht.

8. Zusammensetzung für die Verwendung bei der Behandlung von Krankheiten des Mundraums nach Anspruch 1,
bei der der Photosensibilisator durch einen Bestrahlungsschritt bei vorbestimmten Wellenlängen zwischen 630 und 800 nm aktiviert wird.

9. Zusammensetzung für die Verwendung bei der Behandlung von Krankheiten des Mundraums gemäß Anspruch 8,
bei der die Formulierung für die verzögerte Freisetzung eine Konzentration eines Photosensibilisators aufweist, die die wiederholte Bestrahlung in vorbestimmten Intervallen ohne zusätzliche Anwendung der Zusammensetzung gestattet.

10. Zusammensetzung für die Verwendung bei der Behandlung von Krankheiten des Mundraums nach einem der Ansprüche 1 bis 9,
bei der der Photosensibilisator eine bekannte Photosensibilisatorvorstufe ist und bei der die Photosensibilisatorvorstufe eine Aminolävulinsäure ist.

11. Zusammensetzung für die Verwendung bei der Behandlung von Krankheiten des Mundraums nach Anspruch 4 oder 5,
bei der der Photosensibilisator Komplexe von Mikro- oder Nanoteilchen aufweist, um an der Behandlungsstelle eine hohe Konzentration des Photosensibilisators zu erreichen, der durch eine oder mehrere Bestrahlungen aktiviert wird.

12. Zusammensetzung für die Verwendung bei der Behandlung von Krankheiten des Mundraums nach Anspruch 11,
bei der die Komplexe von Mikro- oder Nanoteilchen Komplexe von Dendrimer-Photosensibilisatoren sind.

13. Zusammensetzung für die Verwendung bei der Behandlung von Krankheiten des Mundraums nach einem der Ansprüche 1 bis 3,
bei der der Photosensibilisator einen abgewandelten Photosensibilisatorkomplex aufweist, der über an dem Photosensibilisatorkomplex angebrachte Antikörper verfügt.

14. Zusammensetzung für die Verwendung bei der Behandlung von Krankheiten des Mundraums nach einem der Ansprüche 1 bis 3,
bei der der Photosensibilisator einen abgewandelten Photosensibilisatorkomplex aufweist, der über molekulare Fragmente verfügt, die an dem Photosensibilisatorkomplex angebracht sind und die in der Lage sind, auf Bestandteile der bakteriellen Zellmembran abgerichtet zu sein.

## Revendications

1. Une composition de photosensibilisateur chronorégulée destinée à une utilisation dans le traitement de maladies de la cavité orale comprenant les opérations suivantes :
a. l'administration d'un photosensibilisateur à une zone nécessitant un traitement dans une formulation chronorégulée, ladite formulation chronorégulée libérant de manière contrôlable ledit photosensibilisateur vers des sites de traitement, et
b. l'activation dudit photosensibilisateur par l'irradiation de ladite zone nécessitant un traitement à des longueurs d'onde présélectionnées de façon à réduire des bactéries au niveau de cette zone de traitement,
- dans laquelle ledit photosensibilisateur est une molécule photosensible sélectionnée dans un groupe se composant de porphyrines, chlorines, phéophorbides, bactériophéophorbides, phthalocyanines, naphthalocyanines, thiazines, xanthènes, colorants au pyrylium, psoralènes et quinones,
- comprenant en outre des micro- ou nanoparticules qui contiennent ledit photosensibilisateur, et ladite libération chronorégulée étant obtenue par une libération contrôlée dudit photosensibilisateur desdites micro- ou nanoparticules,
- dans laquelle ledit photosensibilisateur est activé par une irradiation répétée à des intervalles prédéterminés, qui vont d'une période de quelques heures à quelques jours.

2. La composition destinée à une utilisation dans le traitement de maladies de la cavité orale selon la Revendication 1,
pour le traitement de poches parodontales.

3. La composition destinée à une utilisation dans le traitement de maladies de la cavité orale selon la Revendication 1,
pour le traitement de biofilms bactériens sur des dents, sur des implants ou dans des plaies.

4. La composition destinée à une utilisation dans le traitement de maladies de la cavité orale selon la Revendication 1,
dans laquelle lesdites micro- ou nanoparticules sont des particules biodégradables.

5. La composition destinée à une utilisation dans le traitement de maladies de la cavité orale selon l'une quelconque des Revendications 1 à 4,
dans laquelle ladite libération chronorégulée est obtenue par la libération active de manière périodique dudit photosensibilisateur d'une partie desdites micro- ou nanoparticules, ledit procédé de libération active étant sélectionné dans un groupe se composant d'érosion en bloc, de rayonnement, d'ultrasons, d'agitation par l'intermédiaire d'un brossage, de libération biochimique/enzymatique et de libération chimique.

6. La composition destinée à une utilisation dans le traitement de maladies de la cavité orale selon la Revendication 5,
dans laquelle ladite libération par libération chimique s'effectue par l'application d'un solvant.

7. La composition destinée à une utilisation dans le traitement de maladies de la cavité orale selon la Revendication 1,
pour ladite administration dudit photosensibilisateur dans une formulation sélectionnée dans un groupe se composant d'application directe, d'application avec un bain de bouche, d'application avec un gel et d'application avec un gel de durcissement.

8. La composition destinée à une utilisation dans le traitement de maladies de la cavité orale selon la Revendication 1,
dans laquelle ledit photosensibilisateur est activé par une opération d'irradiation à des longueurs d'onde présélectionnées entre 630 et 800 nm.

9. La composition destinée à une utilisation dans le traitement de maladies de la cavité orale selon la Revendication 8,
dans laquelle ladite formulation chronorégulée contient une concentration de photosensibilisateur qui permet une irradiation répétée à des intervalles prédéterminés sans applications additionnelles de ladite composition.

10. La composition destinée à une utilisation dans le traitement de maladies de la cavité orale selon l'une quelconque des Revendications 1 à 9,
dans laquelle ledit photosensibilisateur est un précurseur de photosensibilisateur connu et dans laquelle ledit précurseur de photosensibilisateur est de l'acide aminolévulinique.

11. La composition destinée à une utilisation dans le traitement de maladies de la cavité orale selon la Revendication 4 ou 5,
dans laquelle ledit photosensibilisateur contient des complexes de micro-ou nanoparticules destinées à obtenir des concentrations élevées dudit photosensibilisateur au niveau d'une zone de traitement qui peuvent être activés par une ou plusieurs irradiations.

12. La composition destinée à une utilisation dans le traitement de maladies de la cavité orale selon la Revendication 11,
dans laquelle lesdits complexes de micro- ou nanoparticules sont des complexes dendrimère-photosensibilisateur.

13. La composition destinée à une utilisation dans le traitement de maladies de la cavité orale selon l'une quelconque des Revendications 1 à 3,
dans laquelle ledit photosensibilisateur contient un complexe photosensibilisateur modifié possédant des anticorps fixés audit complexe photosensibilisateur.

14. La composition destinée à une utilisation dans le traitement de maladies de la cavité orale selon l'une quelconque des Revendications 1 à 3,
dans laquelle ledit photosensibilisateur contient un complexe photosensibilisateur modifié possédant des fragments moléculaires fixés audit complexe photosensibilisateur capables de cibler des composants d'une membrane cellulaire bactérienne.
